# EUROPEAN PATENT SPECIFICATION

(11) **EP 1 372 505 B1**
(45) Date of publication and mention of the grant of the patent: **21.06.2006**
(21) Application number: 01923197.6
(22) Date of filing: 06.04.2001
(51) Int. Cl.: A61B 18/14

(54) **ELECTROSURGICAL INSTRUMENT REDUCING THERMAL SPREAD**
EINE WÄRMEAUSBREITUNG VERMINDERNDES, ELEKTROCHIRURGISCHES INSTRUMENT
INSTRUMENT ELECTROCHIRURGICAL REDUISANT LA PROPAGATION THERMIQUE

(43) Date of publication of application: 02.01.2004
(73) Proprietor: Sherwood Services AG, 8200 Schaffhausen (CH)
(72) Inventor: LAWES, Kate, Ryland, Superior, CO 80027 (US); BUYSSE, Steven, Paul, Longmont, CO 80501 (US); DYCUS, Sean, T., Denver, CO 80205 (US); JOHNSON, Kristin, D., Louisville, CO 80027 (US); MOSES, Michael, C., Boulder, CO 80304 (US); SCHECHTER, Dave, A., Longmont, CO 80503 (US); MIHAICHUK, Carolyn, H., Erie, CO 80506 (US); TETZLAFF, Philip, Mark, Superior, CO 80027 (US)
(74) Representative: HOFFMANN EITLE
(86) International application number: PCT/US2001/011230
(87) International publication number: WO 2002/080784

(56) References cited:
- EP-A- 0 853 922
- WO-A-99/12488
- US-A- 4 763 669
- US-A- 5 674 220

## Description

### BACKGROUND

The present invention relates to electrosurgical instruments used for open and endoscopic surgical procedures. More particularly, the present invention relates to a bipolar forceps having an electrode assembly which is designed to limit and/or reduce thermal spread to adjacent tissue structures and reduce the incidence of flashover during activation.

WO 99/12488 discloses in combination the features of the precharacterizing part of claim 1 below.

### Technical Field

A hemostat or forceps is a simple plier-like tool which uses mechanical action between its jaws to constrict tissue and is commonly used in open surgical procedures to grasp, dissect and/or clamp tissue. Electrosurgical forceps utilize both mechanical clamping action and electrical energy to effect hemostasis by heating the tissue and blood vessels to coagulate, cauterize and/or seal tissue.

By utilizing an electrosurgical forceps, a surgeon can either cauterize, coagulate/desiccate tissue and/or simply reduce or slow bleeding by controlling the intensity, frequency and duration of the electrosurgical energy applied to the tissue. Generally, the electrical configuration of electrosurgical forceps can be categorized in two classifications: 1) monopolar electrosurgical forceps; and 2) bipolar electrosurgical forceps.

Monopolar forceps utilize one active electrode associated with the clamping end effector and a remote patient retum electrode or pad which is attached extemally to the patient. When the electrosurgical energy is applied, the energy travels from the active electrode, to the surgical site, through the patient and to the return electrode.

Bipolar electrosurgical forceps utilize two generally opposing electrodes which are generally disposed on the inner facing or opposing surfaces of the end effectors which are, in turn; electrically coupled to an electrosurgical generator. Each electrode is charged to a different electric potential. Since tissue is a conductor of electrical energy, when the end effectors are utilized to clamp, grasp, seal and/or cut tissue therebetween, the electrical energy can be selectively transferred through the tissue.

Over the last several decades, more and more surgeons are complementing traditional open methods of gaining access to vital organs and body cavities with endoscopes and endoscopic instruments which access organs through small puncture-like incisions. Endoscopic instruments are inserted into the patient through a cannula, or port, that has been made with a trocar. Typical sizes for cannulas range from three millimeters to twelve millimeters. Smaller cannulas are usually preferred, which, as can be appreciated, ultimately presents a design challenge to instrument manufacturers who must find ways to make surgical instruments that fit through the cannulas.

Certain surgical procedures require sealing blood vessels or vascular tissue. However, due to space limitations surgeons can have difficulty suturing vessels or performing other traditional methods of controlling bleeding. e.g., clamping and/or tying-off transected blood vessels. Blood vessels, in the range below two millimeters in diameter, can often be closed using standard electrosurgical techniques. If a larger vessel is severed, it may be necessary for the surgeon to convert the endoscopic procedure into an open-surgical procedure and thereby abandon the benefits of laparoscopy.

It is known that the process of coagulating small vessels is fundamentally different than vessel sealing. For the purposes herein the term "coagulation" is defined as a process of desiccating tissue wherein the tissue cells are ruptured and dried. The term "vessel sealing" is defined as the process of liquefying the collagen in the tissue so that the tissue cross-links and reforms into a fused mass. Thus, coagulation of small vessels is sufficient to close them, however, larger vessels need to be sealed to assure permanent closure.

Several journal articles have disclosed methods for sealing small blood vessels using electrosurgery. An article entitled Studies on Coagulation and the Development of an Automatic Computerized Bipolar Coagulator, J. Neurosurg., Volume 75, July 1991, describes a bipolar coagulator which is used to seal small blood vessels. The article states that it is not possible to safely coagulate arteries with a diameter larger than 2 to 2.5 mm. A second article is entitled Automatically Controlled Bipolar Electrocoagulation - "COA-COMP", Neurosurg. Rev. (1984), pp. 187-190, describes a method for terminating electrosurgical power to the vessel so that charring of the vessel walls can be avoided.

With particular respect to vessel sealing, in order to effect a proper seal with larger vessels, two predominant mechanical parameters must be accurately controlled - the pressure applied to the vessel and the gap between the electrodes both of which affect thickness of the sealed vessel. More particularly, accurate application of the pressure is important for several reasons: 1) to oppose the walls of the vessel; 2) to reduce the tissue impedance to a low enough value that allows enough electrosurgical energy through the tissue; 3) to overcome the forces of expansion during tissue heating; and 4) to contribute to the end tissue thickness which is an indication of a good seal. In some instances a fused vessel wall is optimum between 25.4 µm and 152.4 µm (0.001 and 0.006 inches). Below this range, the seal may shred or tear and above this range the lumens may not be properly or effectively sealed.

Numerous bipolar electrosurgical instruments have been proposed in the past for various open and endoscopic surgical procedures. For example, U.S. Patent No. 2,176,479 to Willis, U.S. Patent Nos. 4,005,714 and 4,031,898 to Hiltebrandt, U.S. Patent Nos. 5,827,274, 5,290,287 and 5,312,433 to Boebel et al., U.S. Patent Nos. 4,370,980, 4,552,143, 5,026,370 and 5,116,332 to Lottick, U.S. Patent No. 5,443,463 to Stem et al., U.S. Patent No. 5,484,436 to Eggers et al. and U.S. Patent No. 5,951,549 to Richardson et al., all relate to electrosurgical instruments for coagulating, sealing and/or cutting vessels or tissue.

In EP 0 853 922 bipolar electrosurgical scissors is disclosed, comprising two cutting blades, each having a shearing face and a cutting edge. Cartridge members being made of an insulating material are mounted on each cutting blade of the scissors. A base portion of each cartridge member is elongated and of similar overall size shape as the scissor blade. Electrodes, which are attached to the insulative base portion of the cartridge comprise an elongated surface portion which lies along the cutting edge of the blade.

It has been found that using electrosurgical instruments to seal, cut and/or cauterize tissue may result in some degree of so-called "thermal spread" across adjacent tissue structure. For the purposes herein the term " thermal spread" refers generally to the heat transfer (heat conduction, heat convection or electrical current dissipation) traveling along the periphery of the electrically conductive surfaces. This can also be termed "collateral damage" to adjacent tissue. As can be appreciated, reducing the thermal spread during an electrical procedure reduces the likelihood of unintentional or undesirable collateral damage to surrounding tissue structures which are adjacent to an intended treatment site.

Instruments which include dielectric coatings disposed along the outer surfaces are known and are used to prevent tissue "blanching" at points normal to the activation site. In other words, these coatings are primarily designed to reduce accidental burning of tissue as a result of incidental contact with the outer surfaces end effectors. So far as is known these coating are not designed or intended to reduce collateral tissue damage or thermal spread to adjacent tissue (tissue lying along the tissue plane).

It has also been found that cleaning and sterilizing many of the prior art bipolar instruments is often impractical as electrodes and/or insulation can be damaged. More particularly, it is known that electrically insulative materials, such as plastics, can be damaged or compromised by repeated sterilization cycles which may ultimately effect the reliability of the instrument and cause so-called "flashover." Flashover as used herein relates to a visual anomaly which develops as a result of inconsistent current tracking over the surface of the insulator or insulative coating and/or activation irregularities which may occur when the instrument is repeatedly used during surgery. Put simply, flashover tends to char the surface of the insulate and may effect the life of the instrument and/or the electrode assembly. The effects and industry standards with respect to flashover are discussed in detail in the Annual Book of ASTM Standards, Vol. 10.02, Designations: D495-84; D618; D2303; and D3638.

Several electrosurgical instruments have been introduced which are known to solve many of the aforementioned problems associated with sealing, cutting, cauterizing and/or coagulating differently-sized vessels. Some of these instruments are described in U.S. Patent No. 6,277,117, entitled.OPEN VESSEL SEALING FORCEPS WITH DISPOSABLE ELECTRODES, U.S. Patent No. 6,511,480, entitled OPEN VESSEL SEALING FORCEPS WITH DISPOSABLE ELECTRODES, U.S. Patent No. 6,458,130; and U.S. Patent No. 6,585,735, entitled ENDOSCOPIC BIPOLAR ELECTROSURGICAL FORCEPS.

Thus, a need exists to develop an electrosurgical instrument which can effectively reduce the undesirable effects of thermal spread across tissue structures and effectively reduce the incidence of flashover.

### SUMMARY

The present invention is defined in claim 1 below and generally relates to an open and/or endoscopic electrosurgical instrument which includes electrodes which are electrically and thermally isolated from the remainder of the instrument by a uniquely designed insulating substrate and electrically conductive surface. It is envisioned that the geometric shape of the insulating substrate relative to the geometric shape of the conductive surface contributes to the overall reduction of collateral damage to adjacent tissue structures. The uniquely-designed geometric configuration of the insulating substrate in connection with the chemical characteristics of the insulating substrate also contributes to a reduction in the incidence of flashover.

More particularly, the present invention relates to an electrosurgical instrument which includes opposing end effectors and a handle for effecting movement of the end effectors relative to one another. The instrument includes a housing and a pair of electrodes. Each electrode includes an electrically conductive surface (e.g., which can be dimensioned for sealing, clamping and/or cutting) and an insulating substrate which may be dimensioned to be selectively engageable with the end effectors such that the electrodes reside in opposing relation relative to one another. The dimensions and/or geometric shape of the insulating substrate are different from the dimensions and/or geometric shape of the electrically conductive surface to reduce thermal spread to adjacent tissue structures. The insulating substrate is made from a material having a Comparative Tracking Index of about 300 volts to about 600 volts to reduce the incidence of flashover.

Preferably, the dimensions of the insulating substrate are different from the dimensions of the electrically conductive surface which not only reduces thermal spread to adjacent tissue structures but may contribute to a reduction in the incidence of flashover. Preferably, the second geometric shape of the insulating substrate is different from the first geometric shape of the conductive surface which effectively reduces thermal spread to adjacent tissue structures and may also contribute to a reduction in the incidence of flashover.

In other embodiments, the insulating substrate is mounted to the electrically conductive surface by stamping, by overmolding, by overmolding a stamped plate and/or by overmolding a metal injection molded plate. All of these manufacturing techniques produce an electrode having an electrically conductive surface which is substantially surrounded by an insulating substrate. These uniquely described embodiments described herein are contemplated to effectively reduce the thermal spread to adjacent tissue structures during and/or immediately following activation. Moreover, certain cross section deviations may also contribute to a reduction in the incidence of flashover. The electrically conductive surface may also include a pinch trim which facilitates secure engagement of the electrically conductive surface to the insulating substrate and also simplifies the overall manufacturing process.

In another embodiment, the electrically conductive surface includes an outer peripheral edge which has a radius and the insulator meets the electrically conductive surface along an adjoining edge which is generally tangential to the radius and/or meets along the radius. Preferably, at the interface, the electrically conductive surface is raised relative to the insulator.

The insulating substrate may be made from a plastic or plastic-based material having a Comparative Tracking Index of about 300 volts to about 600 volts. Preferably, the insulating substrate is substrate is made from a group of materials which include Nylon®, Syndiotactic-polystyrene (SPS), Polybutylene Terephthalate (PBT), Polycarbonate (PC), Acrylonitrile Butadiene Styrene (ABS), Polyphthalamide (PPA), Polyimide, Polyethylene Terephthalate (PET), Polyamide-imide (PAI), Acrylic (PMMA), Polystyrene (PS and HIPS), Polyether Sulfone (PES), Aliphatic Polyketone, Acetal (POM) Copolymer, Polyurethane (PU and TPU), Nylon with Polyphenylene-oxide dispersion and Acrylonitrile Styrene Acrylate. Alternatively, a non-ptastic insulating material, e.g., ceramic, may be used in lieu of or in combination with one or more of the above-identified materials to facilitate the manufacturing process and possibly contribute to the overall reduction of thermal spread to adjacent tissue structures

In another embodiment of the present disclosure, the insulating substrate of each electrode includes at least one mechanical interface for engaging a complementary mechanical interface disposed on the corresponding end effector of the instrument. Preferably, the mechanical interface of the substrate includes a detent and the mechanical interface of the corresponding end effector includes a complementary socket for receiving the detent.

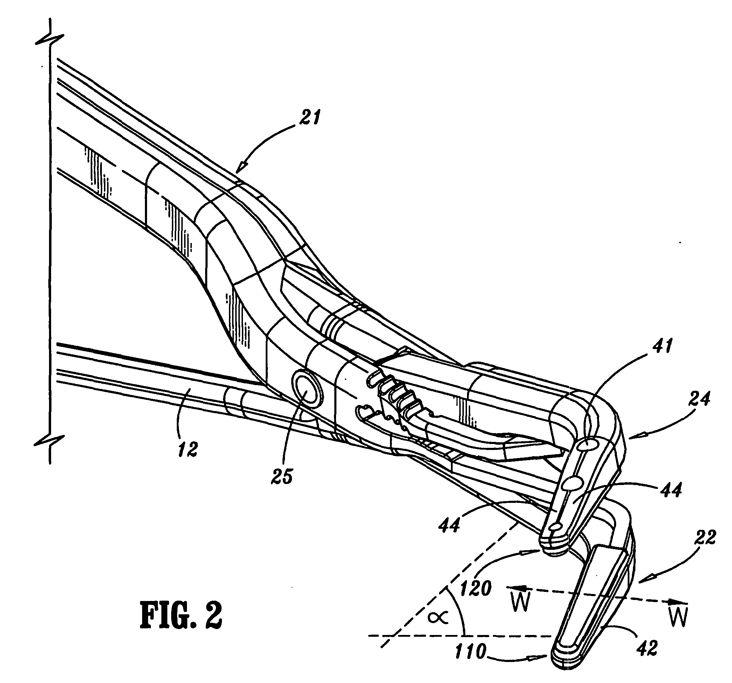

Other embodiments of the present invention include a housing having a bifurcated distal end which forms two resilient and flexible prongs which each carry an electrode designed to engage a corresponding end effector. In another embodiment, the end effectors are disposed at an angle (α) relative to the distal end of the shaft of the electrosurgical instrument. Preferably, the angle is about sixty degrees to about seventy degrees. The end effectors and, in turn, the electrodes, can also be dimensioned to include a taper along a width "W" (See Fig. 2).

The electrode assembly which engages the instrument may be removable, disposable and replaceable after the electrode assembly is used beyond its intended number of activation cycles. Alternatively, the electrode assembly and/or the electrodes may be integrally associated with the end effectors of the instrument and are not removable. In this instance, the electrosurgical instrument (open or endoscopic) may be designed for single use applications and the entire instrument is fully disposable after the surgery is completed.

### BRIEF -DESCRIPTION OF THE DRAWINGS

Fig. 1 is a perspective view of an open electrosurgical instrument according to one embodiment of the present invention;
Fig. 2 is an enlarged, perspective view of a distal end of the electrosurgical instrument shown in Fig. 1;
Fig. 3 is a perspective view with parts separated of the electrosurgical instrument shown in Fig. 1;
Fig. 4 is an enlarged; side view of an electrode assembly of Fig. 1 shown without a cover plate;
Fig. 5 is an enlarged, perspective view of a distal end of the electrode assembly of Fig. 4;
Fig. 6 is a perspective view with parts separated of an upper electrode of the electrode assembly of Fig. 5;
Fig. 7A is a perspective view with parts separated of a lower electrode of the electrode assembly of Fig. 5;
Fig. 7B is a cross section of a prior art electrode configuration with the electrode extending over the sides of the insulator;
Fig. 7C is a cross section of an electrode with the insulator extending beyond the sides of a radiused electrode;
Fig. 7D is a cross section of an overmolded stamped electrode configuration showing the insulator capturing a pinch trim which depends from the electrically conductive surface;
Fig. 7E is a cross section of an electrode configuration showing a compliant barrier disposed about the periphery of the opposing electrodes and insulators which controls/regulates the heat dissipating from the conductive surface.
Fig. 8A is a perspective view of the open forceps of the present instrument showing the operative motion of the electrosurgical instrument about a tubular vessel;
Fig. 8B is a perspective view of an endoscopic version of the present instrument showing the operative motion of the instrument;
Fig. 9 is an enlarged, partial perspective view of a sealing site of a tubular vessel;
Fig. 10 is a longitudinal cross-section of the sealing site taken along line 10-10 of Fig. 9;
Fig. 11 is a longitudinal cross-section of the sealing site of Fig. 9 after separation of the tubular vessel;
Fig. 12 is a contour plot showing the dissipation of the electrosurgical current across the tissue using an electrode without insulation;
Fig. 13A is a contour plot showing the dissipation of the electrosurgical current across the tissue using an electrode with flush insulator;
Fig. 13B is an enlarged contour plot of FIG. 13A showing the current concentration and relative dissipation of the electrosurgical current at an adjoining edge or interface between the insulator and the electrically conductive surface;
Fig. 13C is an enlarged electrical field magnitude plot of the electrode configuration of Fig. 13A showing the current concentration and relative dissipation of the electrosurgical field distribution at an adjoining edge or interface between the insulator and the electrically conductive surface;
Fig. 14A is a contour plot showing the dissipation of the electrosurgical current across the tissue using an electrode with a raised electrically conductive surface and a radiused interface between the electrically conductive surface and the insulator;
Fig. 14B is an enlarged contour plot of FIG. 14A showing the current concentration and relative dissipation of the electrosurgical current at an adjoining edge or interface between the insulator and the electrically conductive surface;
Fig. 14C is an enlarged electrical field magnitude plot of the electrode configuration of Fig. 14A showing the current concentration and relative dissipation of the electrosurgical field distribution at an adjoining edge or interface between the insulator and the electrically conductive surface; and
Fig. 15 is a contour plot showing the dissipation of the electrosurgical current across the tissue using an electrode with a raised electrically conductive surface and a ninety degree (90°) interface between the electrically conductive surface and the insulator.

### DETAILED DESCRIPTION

It has been found that by altering the configuration of the electrode insulating material relative to the electrically conductive surface, surgeons can more readily, more easily and more effectively reduce thermal spread across or to adjacent tissue and/or reduce the incidence of flashover. For the purposes herein the term " thermal spread" refers generally to the heat transfer (heat conduction, heat convection or electrical current dissipation) dissipating along the periphery of the electrically conductive or electrically active surfaces to adjacent tissue. This can also be termed "collateral damage" to adjacent tissue. The term "flashover" is simply a visual anomaly which occurs during activation as a result of inconsistent and/or irregular current tracking over the surface of the insulate which may occur when the instrument is repeatably used during surgery. Flashover tends to char the surface of the insulate and may effect the life of the instrument.

It is envisioned that the configuration of the insulating material which surrounds the perimeter of the electrically conductive surface will effectively reduce current and thermal dissipation to adjacent tissue areas and generally restrict current travel to areas between the opposing electrodes. As mentioned above, this is different from dielectrically coating the outer surfaces of the instrument to prevent tissue "blanching" at points normal to the intended site. These coatings are not designed or intended to reduce collateral tissue damage or thermal spread to adjacent tissue (tissue lying along the tissue activation plane).

More particularly, it is contemplated that altering the geometrical dimensions of the insulator relative to the electrically conductive surface alters the electrical path thereby influencing the thermal spread/collateral damage to adjacent tissue structures. Preferably, the geometry of the insulating substrate also isolates the two electrically opposing poles (i.e., electrodes) from one another thereby reducing the possibility that tissue or tissue fluids can create an unintended bridge or path for current travel. In other words, the insulator and electrically conductive surface are preferably dimensioned such that the current is concentrated between the opposing electrically conductive surfaces as explained in more detail below.

It is also contemplated that one way to reduce the incidence of flashover is to alter the geometry of the insulation relative to the electrically conductive surface which effectively increases the overall distance that the electrical current must travel along the predetermined electrical path. It is also envisioned that manufacturing the insulating substrate from a specific material having certain properties will, likewise, reduce the incidence of flashover during activation.

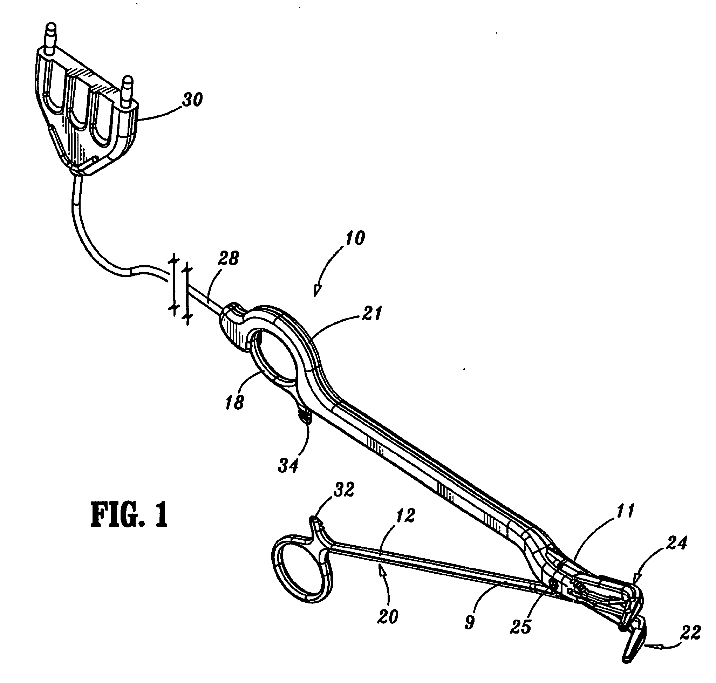

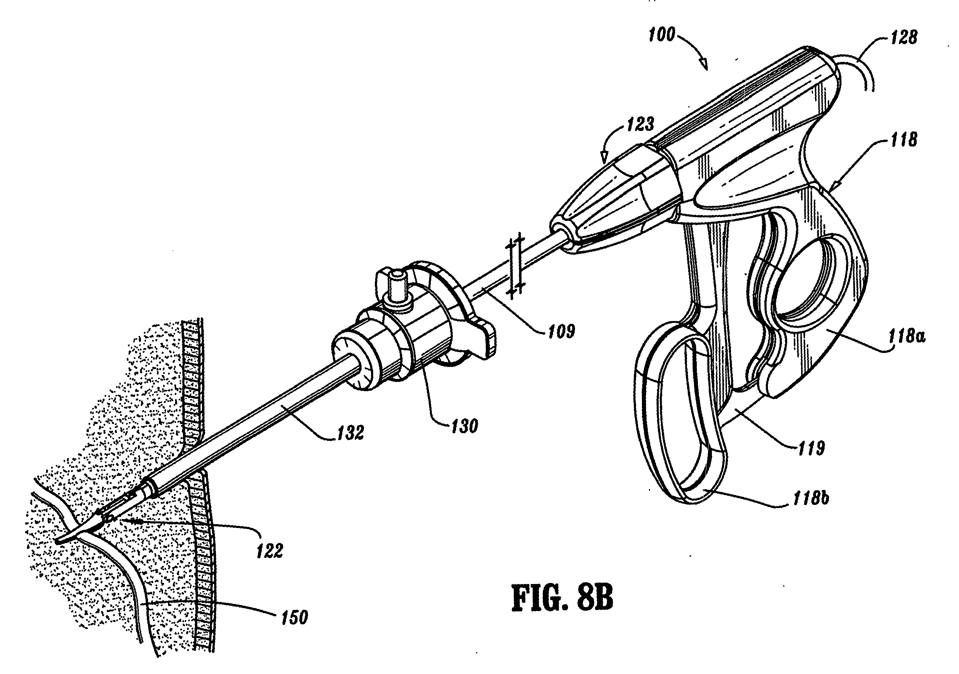

Referring now to Figs. 1-3, a bipolar forceps 10 for use with open surgical procedures is shown by way of example and includes a mechanical forceps 20 and an electrode assembly 21. In the drawings and in the description which follows, the term "proximal", as is traditional, will refer to the end of the forceps 10 which is closer to the user, while the term "distal" will refer to the end which is further from the user. In addition, although the majority of the figures, i.e., Figs 1-7A and 8A, show one embodiment of the presently described instrument for use with open surgical procedures, e.g., forceps 20, it is envisioned that the same properties as shown and described herein may also be employed with or incorporated on an endoscopic instrument 100 such as the embodiment shown by way of example in Fig. 8B.

Figs. 1-3 show mechanical forceps 20 which includes first and second members 9 and 11 which each have an elongated shaft 12 and 14, respectively. Shafts 12 and 14 each include a proximal end 13 and 15 and a distal end 17 and 19, respectively. Each proximal end 13, 15 of each shaft portion 12, 14 includes a handle member 16 and 18 attached thereto which allows a user to effect movement of at least one of the shaft portions, e.g., 12 relative to the other, e.g. 14. Extending from the distal ends 17 and 19 of each shaft portion 12 and 14 are end effectors 24 and 22, respectively. The end effectors 22 and 24 are movable relative to one another in response to movement of handle members 16 and 18.

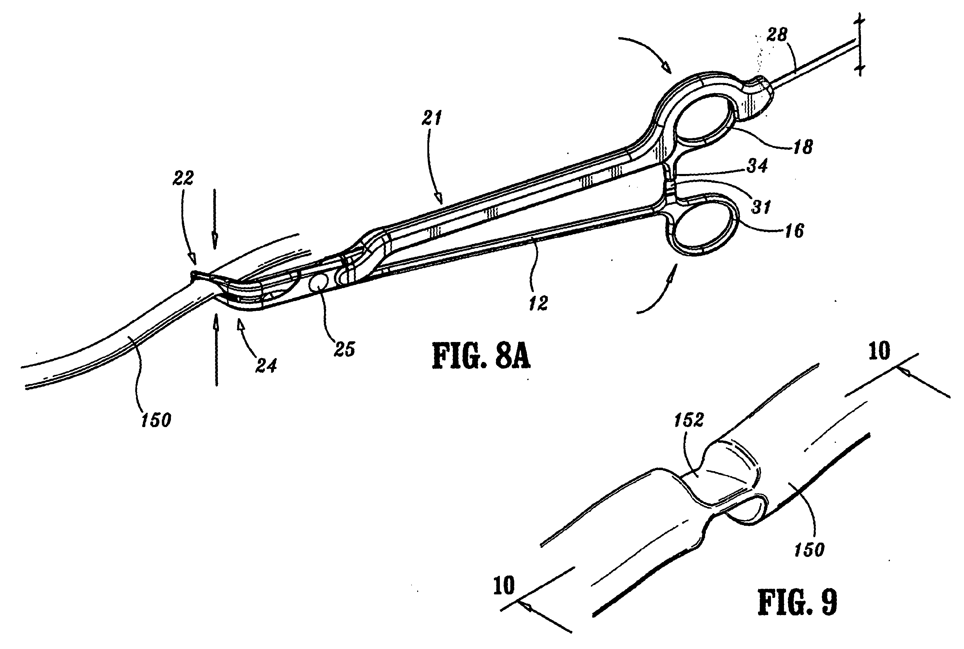

Preferably, shaft portions 12 and 14 are affixed to one another at a point proximate the end effectors 24 and 22 about a pivot 25 such that movement of one of the handles 16, 18 will impart relative movement of the end effectors 24 and 22 from an open position wherein the end effectors 22 and 24 are disposed in spaced relation relative to one another to a closed position wherein the end effectors 22 and 24 incorporate a tubular vessel 150 therebetween (see Figs. 8A and 8B) to effect sealing, cutting or grasping. It is envisioned that pivot 25 has a large surface area to resist twisting and movement of forceps 10 during activation. It is also envisioned that the forceps 10 can be designed such that movement of one or both of the handles 16 and 18 will only cause one of the end effectors, e.g., 24, to move with respect to the other end effector, e.g., 22.

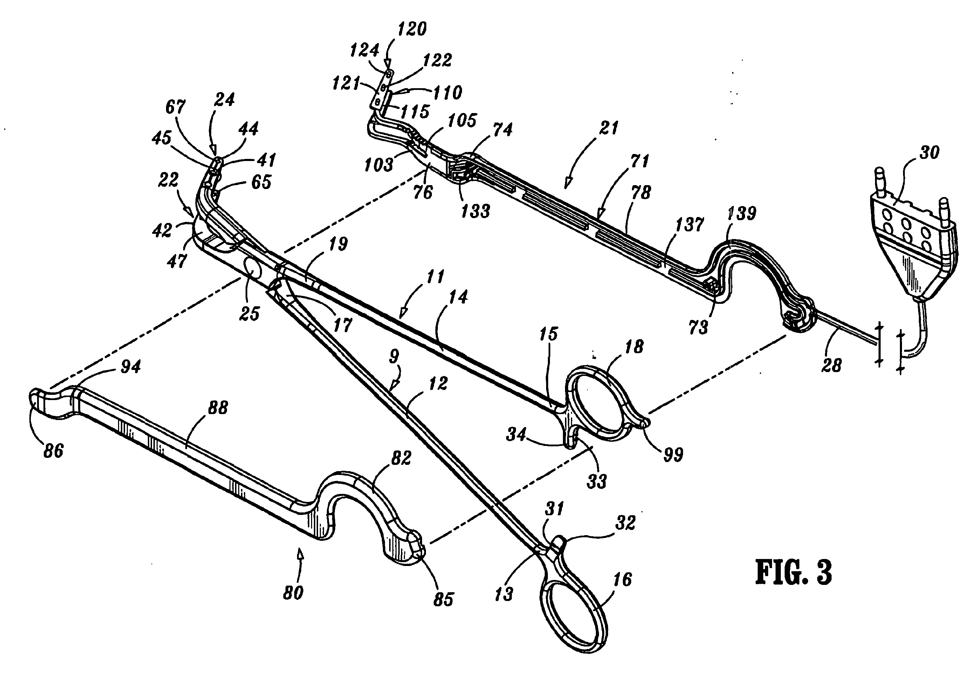

As best seen in Fig. 3, end effector 24 includes an upper or first jaw member 44 which has an inner facing surface 45 and a plurality of mechanical interfaces disposed thereon which are dimensioned to releasable engage a portion of the electrode assembly 21 which will be described in greater detail below. Preferably, the mechanical interfaces include sockets 41 which are disposed at least partially through inner facing surface 45 of jaw member 44 and which are dimensioned to receive a complementary detent 122 attached to upper electrode 120 of the disposable electrode assembly 21. While the term "socket" is used herein, it is contemplated that either a male or female mechanical interface may be used on jaw member 44 with a mating mechanical interface disposed on the electrode assembly 21.

In some cases, it may be preferable to manufacture mechanical interfaces 41 along another side of jaw member 44 to engage a complementary mechanical interface of the electrode assembly 21 in a different manner, e.g., from the side. Jaw member 44 also includes an aperture 67 disposed at least partially through inner face 45 of end effector 24 which is dimensioned to receive a complementary guide pin 124 disposed on electrode 120 of the electrode assembly 21.

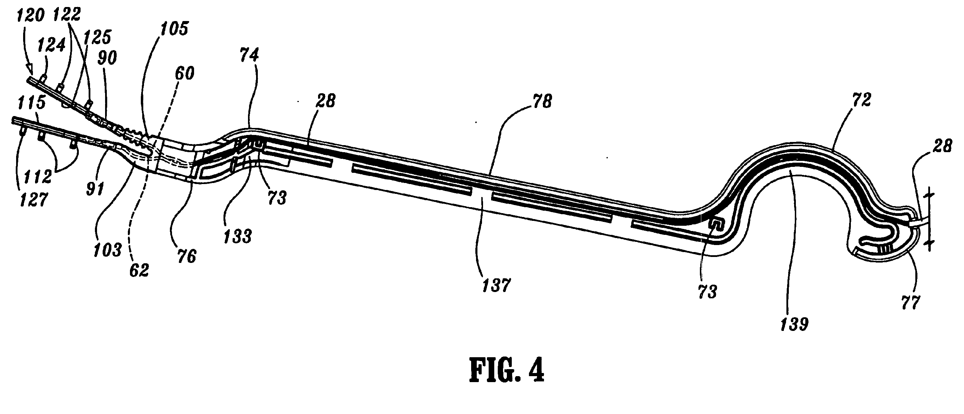

End effector 22 includes a second or lower jaw member 42 which has an inner facing surface 47 which opposes inner facing surface 45. Preferably, jaw members 42 and 44 are dimensioned generally symmetrically, however, in some cases it may be preferable to manufacture the two jaw members 42 and 44 asymmetrically depending upon a particular purpose. In much the same fashion as described above with respect to jaw member 44, jaw member 42 also includes a plurality of mechanical interfaces or sockets 43 disposed thereon which are dimensioned to releasable engage a complementary portion 112 disposed on electrode 110 of the electrode assembly 21 as described below. Likewise, jaw member 42 also includes an aperture 65 disposed at least partially through inner face 47 which is dimensioned to receive a complementary guide pin 127 (see Fig. 4) disposed on electrode 110 of the electrode assembly 21.

Preferably, the end effectors 22, 24 (and, in turn, the jaw members 42 and 44 and the corresponding electrodes 110 and 120) are disposed at an angle alpha (α) relative to the distal ends 19, 17 (See Fig. 2). It is contemplated that the angle alpha (α) is in the range of about 50 degrees to about 70 degrees relative to the distal ends 19, 17. It is envisioned that angling the end effectors 22, 24 at an angle alpha (α) relative to the distal ends 19, 17 may be advantageous for two reasons: 1) the angle of the end effectors, jaw members and electrodes will apply more constant pressure for cutting and/or for a constant tissue thickness at parallel for sealing purposes; and 2) the thicker proximal portion of the electrode, e.g., 110, (as a result of the taper along width "W") will resist bending due to the reaction force of the tissue 150. The tapered "W" shape (Fig. 2) of the electrode 110 is determined by calculating the mechanical advantage variation from the distal to proximal end of the electrode 110 and adjusting the width of the electrode 110 accordingly. It is contemplated that dimensioning the end effectors 22, 24 at an angle of about 50 degrees to about 70 degrees is preferred for accessing and activating specific anatomical structures relevant to prostatectomies and cystectomies, e.g., the dorsal vein complex and the lateral pedicles.

Preferably, shaft members 12 and 14 of the mechanical forceps 20 are designed to transmit a particular desired force to the opposing inner facing surfaces of the of the jaw members 22 and 24, respectively, when clamped or during sealing and/or cutting. In particular, since the shaft members 12 and 14 effectively act together in a spring-like manner (i.e., bending that behaves like a spring), the length, width, height and deflection of the shaft members 12 and 14 will directly effect the overall transmitted force imposed on opposing jaw members 42 and 44. Preferably, jaw members 22 and 24 are more rigid than the shaft members 12 and 14 and the strain energy stored in the shaft members 12 and 14 provides a constant closure force between the jaw members 42 and 44.

Each shaft member 12 and 14 also includes a ratchet portion 32 and 34, respectively. Preferably, each ratchet, e.g., 32, extends from the proximal end 13 of its respective shaft member 12 towards the other ratchet 34 in a generally vertically aligned manner such that the inner facing surfaces of each ratchet 32 and 34 abut one another when the end effectors 22 and 24 are moved from the open position to the closed position. Each ratchet 32 and 34 includes a plurality of flanges 31 and 33, respectively, which project from the inner facing surface of each ratchet 32 and 34 such that the ratchets 32 and 34 can interlock in at least one position. In the embodiment shown in Fig. 1, the ratchets 32 and 34 interlock at several different positions. Preferably, each ratchet position holds a specific, i.e., constant, strain energy in the shaft members 12 and 14 which, in turn, transmits a specific force to the end effectors 22 and 24 and, thus, the electrodes 120 and 110. This is particularly relevant during sealing.

In some cases it may be preferable to include other mechanisms to control and/or limit the movement of the jaw members 42 and 44 relative to one another. For example, a ratchet and pawl system could be utilized to segment the movement of the two handles into discrete units which will, in turn, impart discrete movement to the jaw members 42 and 44 relative to one another.

Preferably, at least one of the shaft members, e.g., 14, includes a tang 99 which facilitates manipulation of the forceps 20 during surgical conditions as well as facilitates attachment of electrode assembly 21 on mechanical forceps 20 as will be described in greater detail below.

As best seen in Figs. 2, 3 and 5, one embodiment of the electrosurgical instrument includes electrode assembly 21 is designed to work in combination with mechanical forceps 20. Preferably, electrode assembly 21 includes housing 71 which has a proximal end 77, a distal end 76 and an elongated shaft plate 78 disposed therebetween. A handle plate 72 is disposed near the proximal end 77 of housing 71 and is sufficiently dimensioned to releasably engage and/or encompass handle 18 of mechanical forceps 20. Likewise, shaft plate 78 is dimensioned to encompass and/or releasably engage shaft 14 and pivot plate 74 disposed near the distal end 76 of housing 71 and is dimensioned to encompass pivot 25 and at least a portion of distal end 19 of mechanical forceps 20. it is contemplated that the electrode assembly 21 can be manufactured to engage either the first or second members 9 and 11 of the mechanical forceps 20 and its respective component parts 12, 16 or 14, 18, respectively.

In the embodiment shown in Fig. 3, handle 18, shaft 14, pivot 25 and a portion of distal end 19 are all dimensioned to fit into corresponding channels located in housing 71. For example, a channel 139 is dimensioned to receive handle 18, a channel 137 is dimensioned to receive shaft 14 and a channel 133 is dimensioned to receive pivot 25 and a portion of distal end 19.

Electrode assembly 21 may also include a cover plate 80 which is designed to encompass and/or engage mechanical forceps 20 in a similar manner as described with respect to the housing 71. More particularly, cover plate 80 includes a proximal end 85, a distal end 86 and an elongated shaft plate 88 disposed therebetween. A handle plate 82 is disposed near the proximal end 85 and is preferably dimensioned to releasable engage and/or encompass handle 18 of mechanical forceps 20. Likewise, shaft plate 88 is dimensioned to encompass and/or releasable engage shaft 14 and a pivot plate 94 disposed near distal end 86 is designed to encompass pivot 25 and distal end 19 of mechanical forceps 20. Preferably, handle 18, shaft 14, pivot 25 and distal end 19 are all dimensioned to fit into corresponding channels (not shown) located in cover plate 80 in a similar manner as described above with respect to the housing 71.

As best seen with respect to Figs. 3 and 4, housing 71 and cover plate 80 are designed to engage one another over first member, e.g., 11, of mechanical forceps 20 such that first member 11 and its respective component parts, e.g., handle 18, shaft 14, distal end 19 and pivot 25, are disposed therebetween. Preferably, housing 71 and cover plate 80 include a plurality of mechanical interfaces disposed at various positions along the interior of housing 71 and cover plate 80 to effect mechanical engagement with one another. More particularly, a plurality of sockets 73 are disposed proximate handle plate 72, shaft plate 78 and pivot plate 74 of housing 71 and are dimensioned to releasably engage a corresponding plurality of detents (not shown) extending from cover plate 80. It is envisioned that either male or female mechanical interfaces or a combination of mechanical interfaces may be disposed within housing 71 with mating mechanical interfaces disposed on or within cover plate 80.

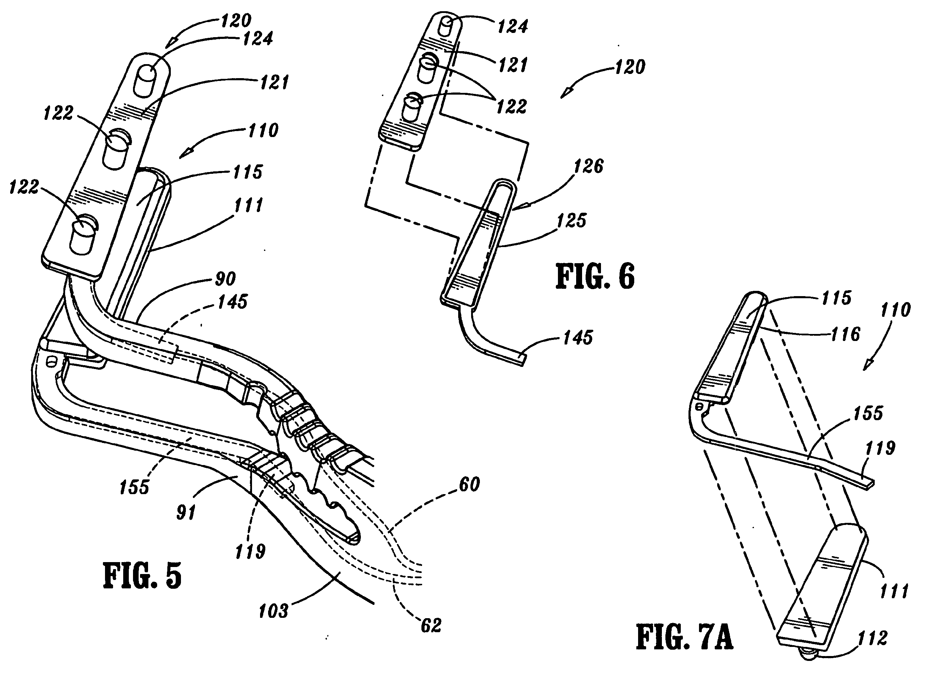

As best seen with respect to Figs. 5-7A, the distal end 76 of electrode assembly 21 is bifurcated such that two prong-like members 103 and 105 extend outwardly therefrom to support electrodes 110 and 120, respectively. More particularly, electrode 120 is affixed at an end 90 of prong 105 and electrode 110 is affixed at an end 91 of prong 103. It is envisioned that the electrodes 110 and 120 can be affixed to the ends 91 and 90 in any known manner, e.g., friction-fit, slide-fit, snap-fit engagement, crimping, etc. Moreover, it is contemplated that the electrodes 110 and 120 may be selectively removable from ends 90 and 91 depending upon a particular purpose and/or to facilitate assembly of the electrode assembly 21. As mentioned above, the inventive concepts disclosed herein may also relate to an electrosurgical instrument which does not include a selectively detachable electrode assembly, but, rather, includes end effectors which have integrally associated electrodes disposed thereon.

A pair of wires 60 and 62 are connected to the electrodes 120 and 110, respectively, as best seen in Figs. 4 and 5. Preferably, wires 60 and 62 are bundled together and form a wire bundle 28 (Fig. 4) which runs from a terminal connector 30 (see Fig. 3), to the proximal end 77 of housing 71, along the interior of housing 71, to distal end 76. Wire bundle 28 is separated into wires 60 and 62 proximate distal end 76 and the wires 60 and 62 are connected to each electrode 120 and 110, respectively. In some cases it may be preferable to capture the wires 60 and 62 or the wire bundle 28 at various pinch points along the inner cavity of the electrode assembly 21 and enclose the wires 60 and 62 within electrode assembly 21 by attaching the cover plate 80.

This arrangement of wires 60 and 62 is designed to be convenient to the user so that there is little interference with the manipulation of bipolar forceps 10. As mentioned above, the proximal end of the wire bundle 28 is connected to a terminal connector 30, however, in some cases it may be preferable to extend wires 60 and 62 to an electrosurgical generator (not shown).

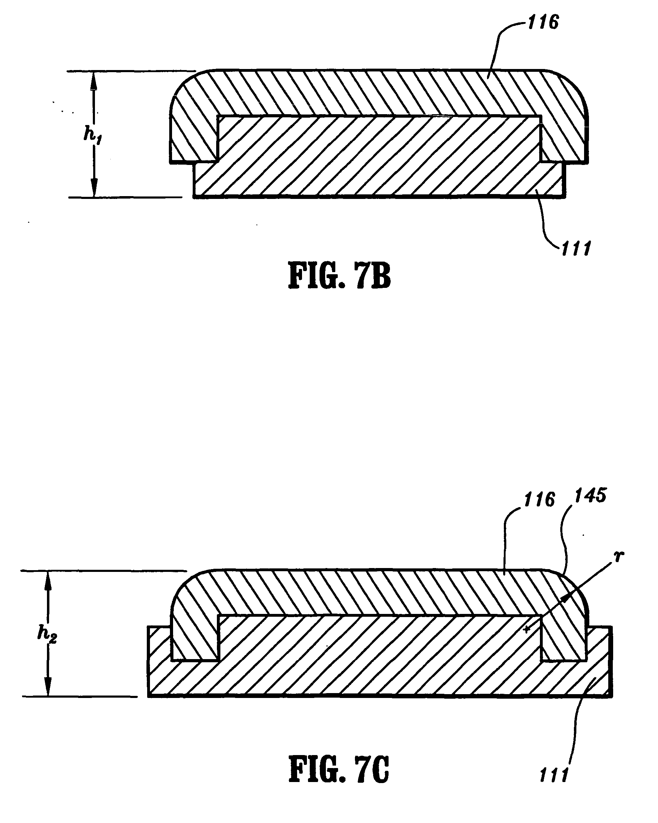

As best seen in Fig. 6, electrode 120 includes an electrically conductive surface 126 and an electrically insulative substrate 121 which are attached to one another by snap-fit engagement or some other method of assembly, e.g., slide-fit, overmolding of a stamping or metal injection molding. Preferably, substrate 121 is made from molded plastic material and is shaped to mechanically engage a corresponding socket 41 located in jaw member 44 of end effector 24 (see Fig. 2). The substrate 121 not only insulates the electric current but it also aligns electrode 120 both of which contribute to the reduction of thermal spread across the tissue and a reduction of the incidence of flashover. Moreover, by attaching the conductive surface 126 to the substrate 121 utilizing one of the above assembly techniques, the alignment and thickness, i.e., height "h2", of the electrode 120 can be controlled. For example and as best illustrated in the comparison of Figs. 7B and 7C , the overmolding manufacturing technique reduces the overall height "h2" (Fig. 7C) of the electrode 120 compared to traditional manufacturing techniques which yield a height of "h1" (Fig. 7B). The smaller height "h2" allows a user access to smaller areas within the body and facilitates activation around more delicate tissue areas.

Moreover, it is contemplated that the overmolding technique provides more insulation along the side of the electrically conductive surface which also reduces thermal spread due to less electrode to tissue contact. It is envisioned that by dimensioning substrate, e.g., 121 and electrode 120 in this fashion (i.e., with reduced conductive surface area), the current is restricted (i.e., concentrated) to the intended area rather than current traveling to tissue outside the intended area which may come into contact with an outer edge of the electrode 120 (see Fig. 7B). Providing more insulation along the side of the electrically conductive surface may also effectively reduces the incidence of flashover.

Preferably, substrate 121 includes a plurality of bifurcated detents 122 which are shaped to compress during insertion into sockets 41 and expand and releasably engage sockets 41 after insertion. It is envisioned that snap-fit engagement of the electrode 120 and the jaw member 44 will accommodate a broader range of manufacturing tolerances. Substrate 121 also includes an alignment or guide pin 124 which is dimensioned to engage aperture 67 of jaw member 44. A slide-fit technique is also contemplated such as the slide-fit technique described with respect to commonly-assigned, co-pending U.S. Application Serial No. PCT/US01/11218, filing date April 6, 2001, by Tetzlaff et al.

Conductive surface 126 includes a wire crimp 145 designed to engage the distal end 90 of prong 105 of electrode assembly 21 and electrically engage a corresponding wire connector affixed to wire 60 located within electrode assembly 21. Conductive surface 126 also includes an opposing face 125 which is designed to conduct an electrosurgical current to a tubular vessel or tissue 150 when it is held thereagainst. It is envisioned that the conductive surfaces 126 (116) may be dimensioned as a sealing surface, a clamping surface and/or a shearing or cutting surface depending upon a particular purpose.

Electrode 110 includes similar elements and materials for insulating and conducting electrosurgical current to tissue 150. More particularly, electrode 110 includes an electrically conductive surface 116 and an electrically insulative substrate 111 which are attached to one another by one of the above methods of assembly. Substrate 111 includes a plurality of detents 112 which are dimensioned to engage a corresponding plurality of sockets 43 and aperture 65 located in jaw member 42. Conductive surface 116 includes an extension 155 having a wire crimp 119 which engages the distal end 91 of prong 103 and electrically engages a corresponding wire connector affixed to wire 62 located in housing 71. Conductive surface 116 also includes an opposing face 115 which conducts an electrosurgical current to a tubular vessel or tissue 150 when it is held thereagainst. It is contemplated that electrodes 110 and 120 can be formed as one piece and include similar components and/or dimensions for insulating and conducting electrical energy in a manner to effectively reduce thermal spread and the incidence of flashover. Stray current may be further restricted by casting the forceps and/or manufacturing the forceps using a non-conductive material and/or coating the edges of the electrodes 110 and 120 with an insulative coating.

As mentioned above, it is envisioned that flashover and thermal spread may be reduced by altering the physical dimensions (geometry/shape) of the insulators and/or the chemical characteristics of the insulators. With particular respect to thermal spread, it is envisioned that manufacturing the electrodes 110 and 120 in this fashion will reduce thermal spread and stray currents that may travel to the electrosurgical instrument. More particularly, the varying geometry of the insulator 111 compared to the electrically conductive surface 116 also isolates the two opposing poles during activation thereby reducing the possibility that tissue or fluids will bridge a path for stray current travel to surrounding tissue. With respect to flashover, altering the geometry of the insulator 11 and/or conductive surface creates a longer path for the current to travel over the insulator 111 before flashover occurs.

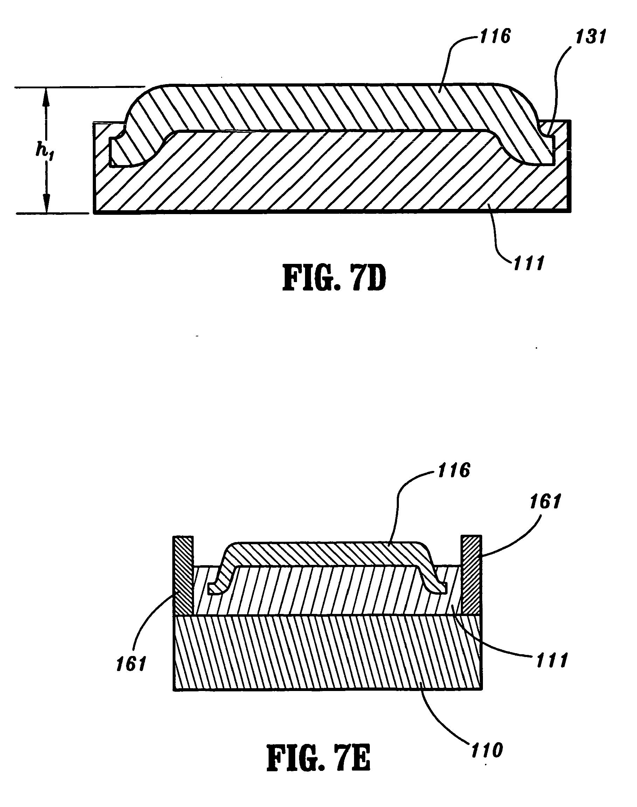

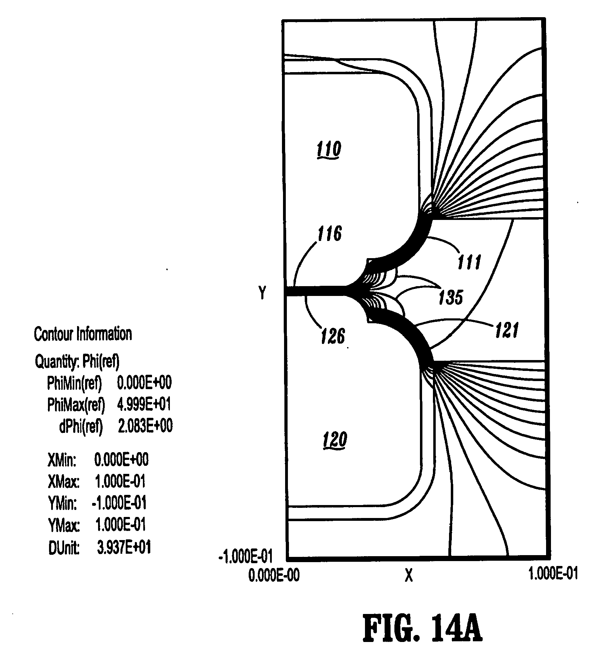

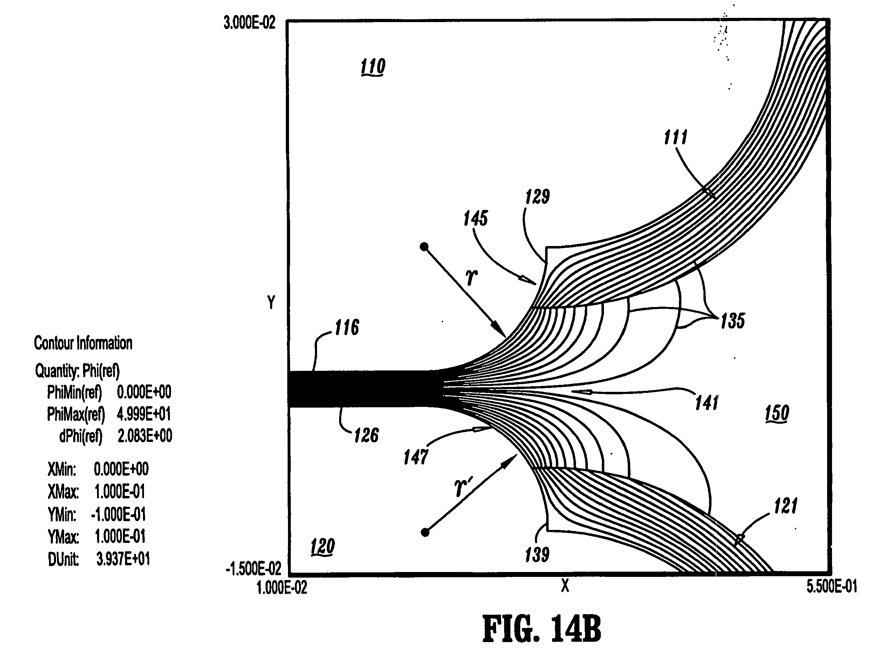

For example and as best shown in the comparison of Fig. 7B (prior art) with newly disclosed Figs. 7C, 7D, 14A and 14B substrates 111, 121 are designed to extend along width "W" (Fig. 2) such that the width of the insulating substrate, e.g., 111, exceeds the width of the electrically conductive surface, e.g., 116. It is envisioned that these electrically conductive surface 116 and insulator 111 configurations may be accomplished by various manufacturing techniques such as overmolding of a stamping and/or metal injection molding. Stamping is defined herein to encompass virtually any press operation known in the trade, including, but not limited to: blanking, shearing, hot or cold forming, drawing, bending and coining. Other manufacturing techniques may also be employed to achieve similar electrically conductive surface 116 and insulator 111 configurations which will effectively reduce thermal spread to adjacent tissue.

As best seen in Fig. 7D, the electrode 116 may also include a pinch trim 131 which facilitates secure, integral engagement of the insulate 111 and the electrically conductive surface 116 during the assembly and/or manufacturing process. FIG. 7E shows another embodiment of the present invention wherein a compliant material 161 is disposed about the outer peripheries of the electrically conductive surfaces 116, 126 and the substrates 111, 121. it is envisioned that the compliant material 161 acts as a mechanical barrier by restricting heat and steam emanating from the surface thereby reduces thermal spread to surrounding tissue. One or more barriers 161 may be attached to the end effectors 22, 24 and/or the insulting substrate 111, 121 depending upon a particular purpose of to achieve a particular result.

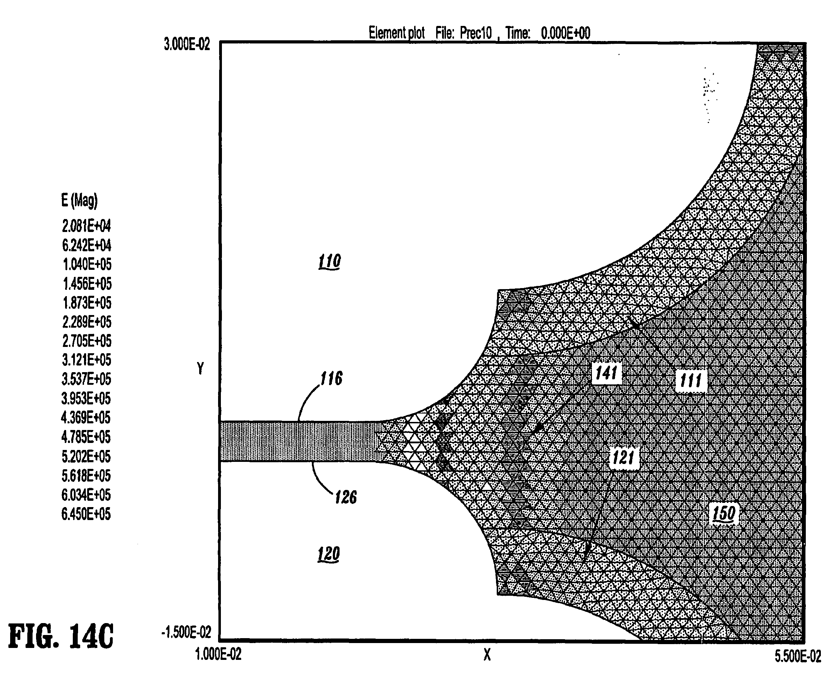

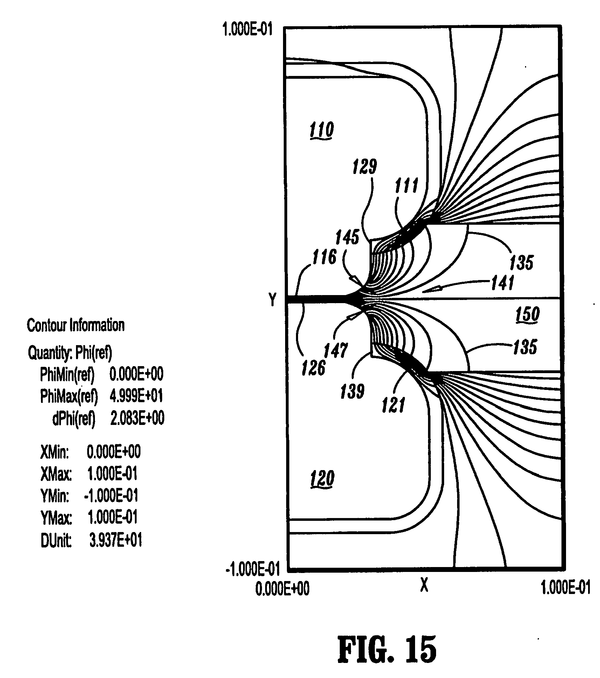

Figs. 14A, 14B, 14C and 15 show the electrically conducive surfaces 116, 126 raised relative to the insulative coatings or insulators 111, 121. Preferably, the electrically conductive surface 116, 126 is radiused or curved which reduces current concentration and the dissipation of stray currents to surrounding tissue structures. It is contemplated that the insulators 111, 121 and electrically conductive surfaces 116, 126 can be dimensioned to meet at or generally along interfaces or adjoining longitudinally-oriented edges 129, 139 which are radiused to reduce current concentrations 141 and current dissipation proximate the interfaces 129, 139 and opposing electrically conductive surfaces 116, 126.

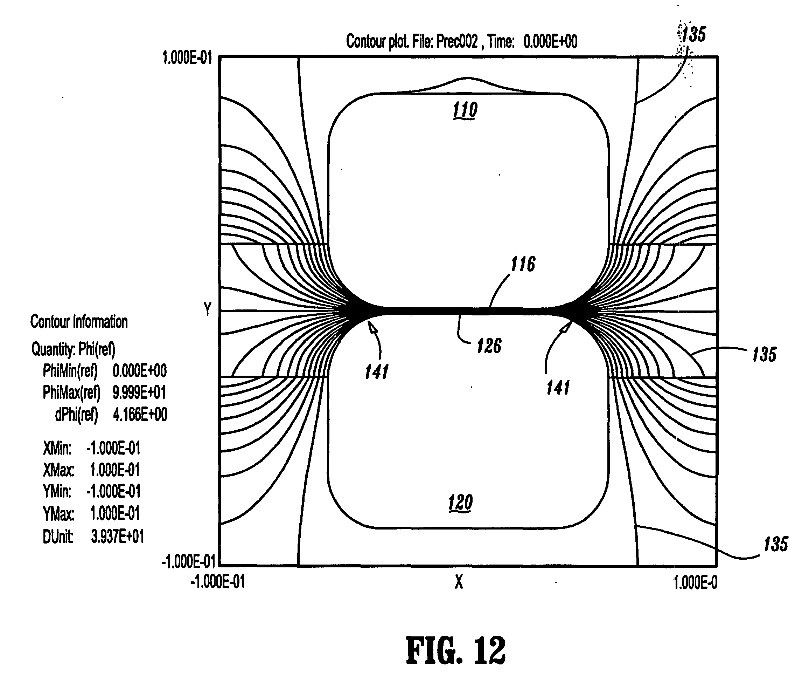

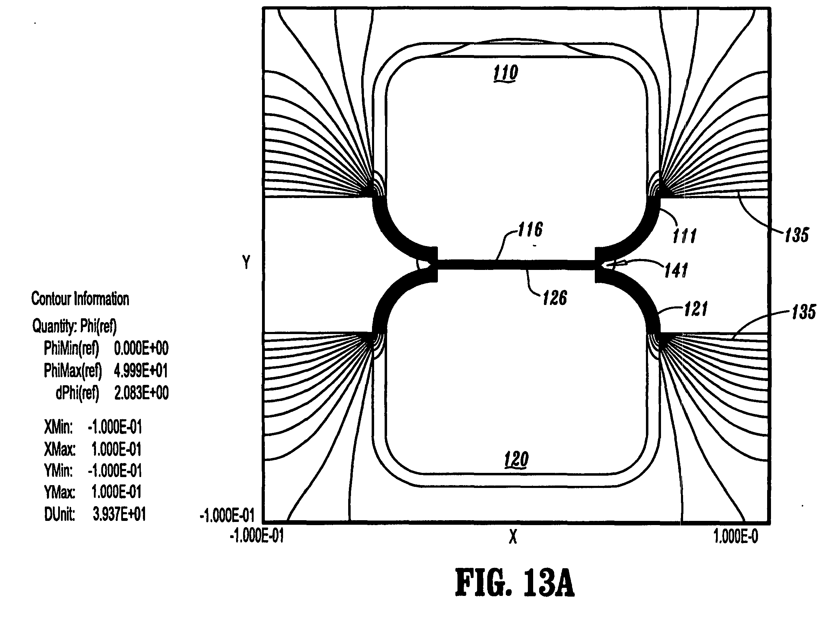

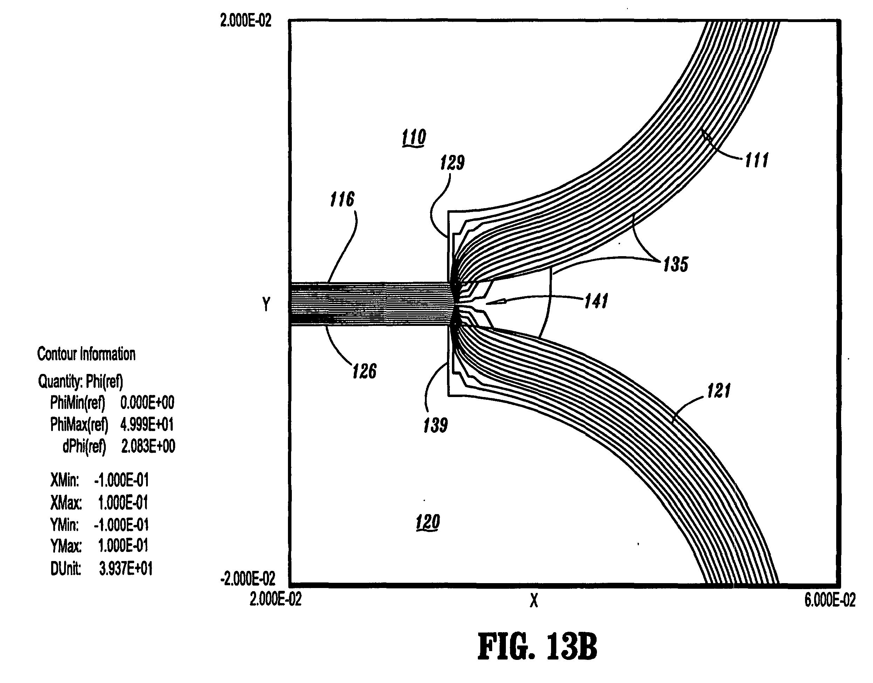

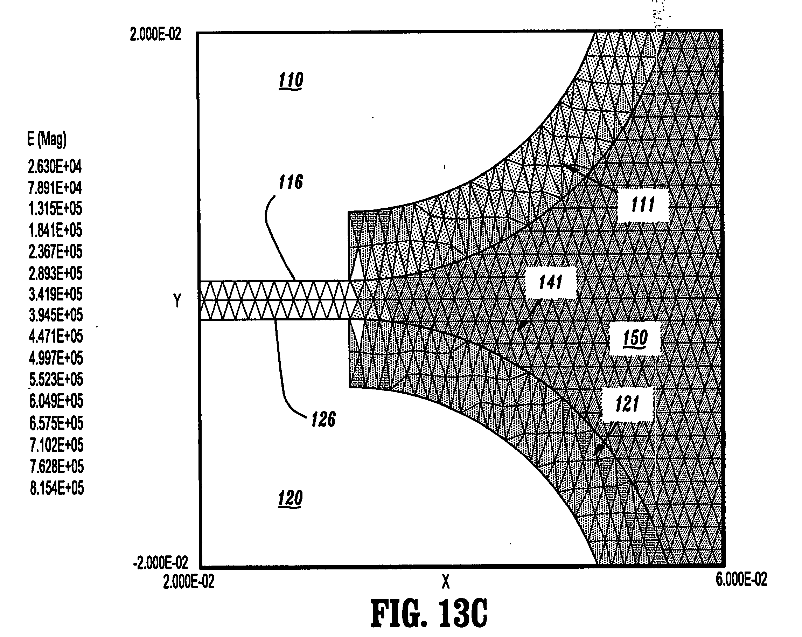

For example and by way of illustration, Figs. 12 and 13A-13C show other electrode 110, 120 configurations which are known in the prior art. Fig. 12 shows an example of uninsulated (i.e., without insulators 111, 121) opposing electrodes 110, 120 during activation illustrating the electrical field distribution 135 emanating from the opposing electrically conductive surfaces 116, 126 (it is known that current flows perpendicular to these electrical field lines). As can be appreciated, the electrical field 135 emanates well beyond the intended treatment site which can contribute to increased collateral tissue damage and possibly cutting.

By providing insulators 111, 121 which are flush with the electrically conductive surfaces 116, 126 as shown in Figs. 13A-13C, the electrical field distribution 135 can be significantly reduced. However, as the enlarged views of Figs. 13B and 13C illustrate, a current concentration 141 tends to develop between opposing electrically conductive surfaces 116, 126 and at or proximate interfaces 129, 139. This current concentration 141 may also lead to negative effects and possibly cause cutting of the tissue or sticking of the tissue to the electrode or electrically conductive surfaces at this site.

Figs. 14A-15 show various electrode 110, 120 configurations according to the present invention in which the electrically conductive surfaces 116, 126 and the insulators 111, 121. are designed to reduce the amount of current concentration 141 between opposing electrodes 110, 120. More particularly, Figs. 14A and 14B show a pair of raised electrically conductive surfaces 116, 126 (relative to the insulators 111, 121) which include outer peripheries 145, 147 having radii "r" and "r' ", respectively. Preferably, insulators 111, 121 meet outer peripheries 145, 147 and form adjoining edges or interfaces 129, 139 which track along radii "r" and "r' ", respectively. It is contemplated that configuring the electrodes 110, 120 in this manner will effectively reduce the current concentration 141 between the outer peripheries 145, 147 of the opposing electrically conductive surfaces 116, 126. As can be appreciated, configuring the electrically conductive surfaces 116, 126 and insulators 111, 121 with this unique profile, additionally provides a more uniform, consistent and more easily controllable electrical field distribution 135 across the adjacent tissue structures. Turning back to Fig. 7C, it is envisioned that insulator 111 may also meet outer periphery 145 in a generally tangential fashion about radius "r". Again, this profile also tends to reduce current concentration and thermal spread and may also contribute to a reduction in the incidence of flashover.

Fig. 15 also shows the insulators 111, 121 and the electrically conductive surfaces 116, 126 meeting at an angle of ninety degrees (90°), however, the insulator 111, 121 is positioned further from the radiused edge 145 of the electrically conductive surface 116, 126. It is envisioned that too much exposure of the edge 145 may initiate the formation of new and/or additional stray currents or electrical fields proximate the interface 129, 139 thereby nullifying the benefits of manufacturing the surface 116, 126 with a radiused edge 145.

Preferably, the radius "r" and "r' " of the outer peripheries 145, 147 of the electrically conductive surface are about the same and are about 2.54 µm (ten thousandths of an inch) to about 0.85 µm (thirty thousandths of an inch). However, it is contemplated that each radii "r" and "r'" may be sized differently depending upon a particular purpose or to achieve a desired result.

Although it is contemplated that geometric modification of the insulator 11 relative to the electrically conductive sealing surface 116 reduces the incidence of flashover and thermal spread, in some cases it may be preferable to simply utilize a different material for the insulator to reduce flashover and thermal spread. For example and with particular respect to flashover, it is known that all plastics have a different resistance to flashover which is commonly measured using a Comparative Tracking Index (CTI). The CTI value required to resist flashover is typically dictated in part by the maximum voltage of the electrosurgical generator, however, other parameters such as frequency also typically effect flashover.

It has been found that in lieu of or in addition to changing the geometry of the insulator 111 and/or conductive surface 116, a plastic insulation can be employed having a CTI value of about 300 to about 600 volts. Examples of high CTI materials include Nylon® and syndiotactic polystyrenes such as QUESTRA® manufactured by DOW Chemical. Other materials may also be utilized either alone or in combination to reduce flashover, e.g., Nylon®, Syndiotactic-polystryrene (SPS), Polybutylene Terephthalate (PBT), Polycarbonate (PC), Acrylonitrile Butadiene Styrene (ABS), Polyphthalamide (PPA), Polymide, Polyethylene Terephthalate (PET), Polyamide-imide (PAI), Acrylic (PMMA), Polystyrene (PS and HIPS), Polyether Sulfone (PES), Aliphatic Polyketone, Acetal (POM) Copolymer, Polyurethane (PU and TPU), Nylon® with Polyphenylene-oxide dispersion and Acrylonitrile Styrene Acrylate.

In some cases, however, it may be preferable to alter both the geometry of the insulator 111 and/or conductive surface 116 and/or utilize a plastic insulation that does not have a CTI value of about 300 to about 600 volts. Alternatively, certain coatings can be utilized either alone or in combination with one of the above manufacturing techniques to reduce flashover and thermal spread.

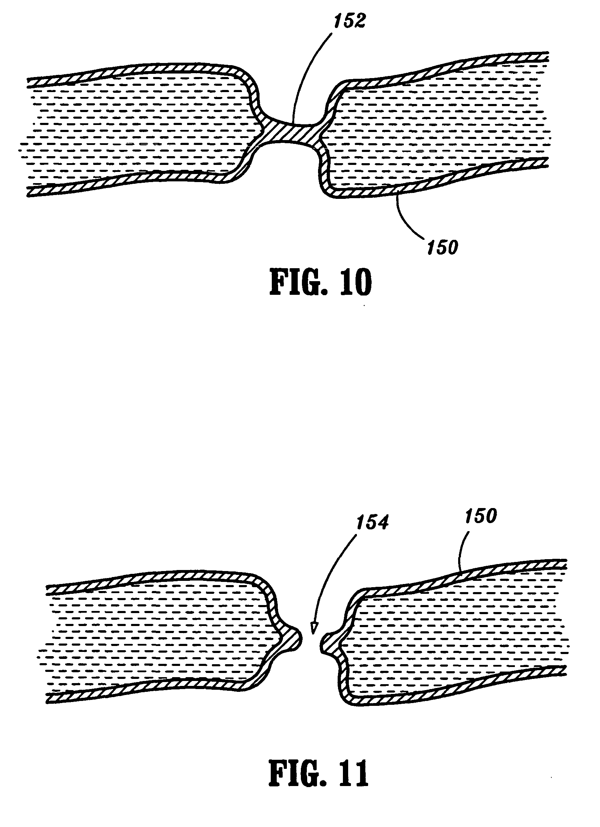

Fig. 8A shows one embodiment of the present invention which shows a bipolar forceps 10 during use wherein the handle members 16 and 18 are moved closer to one another to apply clamping force to the tubular tissue 150 to effect a seal 152 as shown in Figs. 9 and 10. Once sealed, the tubular vessel 150 can be cut along seal 152 to separate the tissue 150 and form a gap 154 therebetween as shown in Fig. 11. Alternatively, the electrically conductive surfaces 116, 126, electrodes 110, 120 and/or the jaw members 42, 44 may be dimensioned as shearing surfaces which effectively cut the tissue when the jaw members 42, 44 are moved relative to one another.

After the bipolar forceps 10 is used or if the electrode assembly 21 is damaged, the electrode assembly 21 can be easily removed and/or replaced and a new electrode assembly 21 may be attached to the forceps in a similar manner as described above. It is envisioned that by making the electrode assembly 21 disposable, the electrode assembly 21 is less likely to become damaged since it is only intended for a single operation and, therefore, does not require cleaning or sterilization. As a result, the functionality and consistency of the components, e.g., the electrically conductive surfaces 126,116 and insulating surfaces 121, 111, will assure a reliable reduction of thermal spread across tissue and/or reduce the incidence of flashover. Alternatively, the entire electrosurgical instrument may be disposable which, again, may contribute to a reduction of thermal spread across tissue and/or reduce the incidence of flashover

Fig. 8B shows an endoscopic bipolar instrument 100 during use wherein movement of a handle assembly 128 applies clamping force on the tubular tissue 150 to effect a seal 152 as shown in Figs. 9-11. As shown, a shaft 109 and the electrode assembly 122 are inserted through a trocar 130 and cannula 132 and a handle assembly 118 is actuated to cause opposing jaw members of the electrode assembly 122 to grasp tubular vessel 150 therebetween. More particularly, a movable handle 118b is moved progressively towards a fixed handle 118a which, in turn, causes relative movement of the jaw members from an open, spaced-apart position to a closed, activation position. A rotating member 123 allows the user to rotate the electrode assembly 122 into position about the tubular tissue 150 prior to activation. Again, the electrically conductive surfaces 116, 126, electrodes 110, 120 and/or the jaw members 42, 44 may be dimensioned as shearing surfaces which effectively cut the tissue when the jaw members 42, 44 are moved relative to one another.

After the jaw members are closed about the tissue 150, the user then applies electrosurgical energy via connection 128 to the tissue 150. By controlling the intensity, frequency and duration of the electrosurgical energy applied to the tissue 150, the user can either cauterize, coagulate/desiccate, seal, cut and/or simply reduce or slow bleeding with minimal collateral or thermal damage to surrounding tissue and with minimal incidence of flashover.

From the foregoing and with reference to the various figure drawings, those skilled in the art will appreciate that certain modifications can also be made to the present disclosure without departing from the scope of the present disclosure. For example, although it is preferable that electrodes 110 and 120 meet in parallel opposition, and, therefore, meet on the same plane, in some cases it may be preferable to slightly bias the electrodes 110 and 120 to meet each other at a distal end such that additional closure force on the handles 16 and 18 is required to deflect the electrodes in the same plane.

It is envisioned that the outer surface of the end effectors may include a nickel-based material, coating, stamping, metal injection molding which is designed to reduce adhesion between the end effectors (or components thereof) with the surrounding tissue during activation.

While only one embodiment of the invention has been described, it is not intended that the invention be limited thereto. Therefore, the above description should not be construed as limiting, but merely as exemplifications of a preferred embodiment. Those skilled in the art will envision other modifications within the scope of the claims appended hereto.

## Claims

1. Electrosurgical forceps (20, 100) having opposing end effectors (22, 44) and a handle (18, 118) for effecting movement of the end effectors relative to one another, comprising:
a housing (71);
a pair of electrodes (110, 120) each including an electrically conductive surface (116, 126) and an insulating substrate (111, 121);
**characterized in that**
the dimensions and/or geometric shape of the insulating substrate are different from the dimensions and/or geometric shape of the electrically conductive surface to reduce thermal spread to adjacent tissue structures;
the insulating substrate is made from a material having a Comparative Tracking Index of about 300 volts to about 600 volts to reduce the incidence of flashover; and
a compliant material (161) is disposed about an outer peripheral edge of the electrically conductive surface and the insulating substrate.

2. Forceps according to claim 1, wherein the outer peripheral edge of at least one electrically conductive surface includes a radius and the insulator meets the electrically conductive surface along an adjoining edge which is along the radius.

3. Forceps as claimed in claim 1 or 2, wherein the material of the insulating substrate is a polyamide or a polystyrene.

4. Forceps according to any one of the preceding claims, wherein the insulating substrate is mounted to the electrically conducive surface by overmolding a stamped plate.

5. Forceps according to any one of claims 1 to 3, wherein the insulating substrate is mounted to the electrically conductive surface by overmolding a metal injection molded plate.

6. Forceps according to any one of the preceding claims, wherein the electrically conductive surface is raised relative to the insulator.

7. Forceps according to any one of the preceding claims, wherein the electrically conductive surface of at least one electrode includes a pinch trim (131) and the insulating substrate extends beyond the periphery of the electrically conductive surface.

8. Forceps according to any one of the preceding claims, wherein the insulating substrate of each of the electrodes includes at least one mechanical interface for engaging a complementary mechanical interface disposed on the corresponding end effector of the instrument.

9. Forceps according to claim 8, wherein the mechanical interface of at least one of the substrates includes at least one detent (112, 122) and the mechanical interface of the corresponding end effector includes at least one complementary socket (41, 43) for receiving the detent.

10. Forceps according to any one of the preceding claims, wherein the electrically conductive surfaces of the opposing jaw members cooperate to seal tissue.

11. Forceps according to any one of the preceding claims, wherein the electrically conductive surfaces of the opposing jaw members include shearing surfaces which cooperate to cut tissue.

12. Forceps according to any one of the preceding claims, wherein the housing includes a bifurcated distal end which forms two prongs and each prong is removably attached to one of the end effectors.

13. Forceps according to any one of the preceding claims, wherein at least one of the opposing end effectors and opposing electrodes is tapered.

14. Forceps according to any one of the preceding claims, wherein the end effectors are disposed at an angle (α) relative to a shaft of the electrosurgical instrument.

15. Forceps according to claim 14, wherein the angle is about sixty degrees to about seventy degrees.

16. Forceps according to any one of the preceding claims, wherein the insulating substrate and the electrically conductive surface meet at an angle of ninety degrees.

## Patentansprüche

1. Elektrochirurgische Zange (20, 100), die gegenüberliegende Endeffektoren (22, 44) und einen Griff (18, 118) zum Bewirken einer Relativbewegung der Endeffektoren zueinander aufweist, umfassend:
ein Gehäuse (71);
ein Paar von Elektroden (110, 120), jeweils umfassend eine elektrisch leitende Oberfläche (116, 126) und ein isolierendes Substrat (111, 121);
**dadurch gekennzeichnet, dass**
die Dimensionen und/oder geometrische Form des Isoliersubstrats verschieden von den Dimensionen
und/oder der geometrischen Form der elektrisch leitenden Oberfläche ist, um die thermische Ausbreitung auf angrenzende Gewebestrukturen zu reduzieren;
wobei das Isolationssubstrat aus einem Material gemacht ist, das einen Vergleichsnachführindex (Comparative Tracking Index) von ungefähr 300 Volt bis ungefähr 600 Volt aufweist, um das Auftreten von Funkenüberschlägen zu reduzieren; und
ein nachgiebiges Material (161) ist über eine äußere Umfangskante der elektrisch leitenden Oberfläche und des Isolationssubstrates hinweg angeordnet.

2. Zange gemäß Anspruch 1, wobei die äußere Umfangskante zumindest einer elektrisch leitenden Oberfläche einen Radius umfasst und der Isolator die elektrisch leitende Oberfläche entlang einer angrenzenden Kante trifft, die sich entlang des Radius erstreckt.

3. Zange gemäß Anspruch 1 oder 2, wobei das Material des Isolationssubstrates ein Polyamid oder ein Polystyren ist.

4. Zange gemäß einem der vorstehenden Ansprüche, wobei das Isolationssubstrat an der elektrisch leitenden Oberfläche durch Überformen einer ausgestanzten Platte befestigt ist.

5. Zange gemäß einem der Ansprüche 1 - 3, wobei das Isolationssubstrat an der elektrisch leitenden Oberfläche durch Überformen einer metallischen, spritzgegossenen Platte befestigt ist.

6. Zange gemäß einem der vorstehenden Ansprüche, wobei die elektrisch leitende Oberfläche gegenüber dem Isolator erhoben ist.

7. Zange gemäß einem der vorstehenden Ansprüche, wobei die elektrisch leitende Oberfläche zumindest einer Elektrode eine Abdrückungseinstellung (131) umfasst und sich das Isoliersubstrat über den Umfang der elektrisch leitenden Oberfläche hinaus erstreckt.

8. Zange gemäß einem der vorstehenden Ansprüche, wobei das Isoliersubstrat jeder der Elektroden zumindest eine mechanische Kopplungsfläche zum Eingriff einer komplementären mechanischen Kopplungsstelle aufweist, die an dem korrespondierenden Endeffektor des Instruments angeordnet ist.

9. Zange gemäß Anspruch 8, wobei die mechanische Kopplungsstelle zumindest eines der Substrate zumindest eine Raste (112, 122) umfasst und die mechanische Kopplungsstelle des korrespondierenden Endeffektors zumindest einen komplementären Sockel (41, 43) zur Aufnahme der Raste umfasst.

10. Zange gemäß einem der vorstehenden Ansprüche, wobei die elektrisch leitenden Oberflächen der gegenüberliegenden Backenteile miteinander kooperieren, um Gewebe abzudichten.

11. Zange gemäß einem der vorstehenden Ansprüche, wobei die elektrisch leitenden Oberflächen der gegenüberliegenden Backenteile Scheroberflächen umfassen, die miteinander kooperieren, um Gewebe zu schneiden.

12. Zange gemäß einem der vorstehenden Ansprüche, wobei das Gehäuse ein gegabeltes distales Ende umfasst, das zwei Zinken ausformt und jede Zinke mit einem der Endeffektoren entnehmbar verbunden ist.

13. Zange gemäß einem der vorstehenden Ansprüche, wobei zumindest einer der gegenüberliegenden Endeffektoren und der gegenüberliegenden Elektroden spitz zuläuft.

14. Zange gemäß einem der vorstehenden Ansprüche, wobei die Endeffektoren in einem Winkel (α) sind relativ zu einem Griff des elektrochirurgischen Instruments zueinander angeordnet.

15. Zange gemäß Anspruch 14, wobei der Winkel ungefähr 60° bis ungefähr 70° ist.

16. Zange gemäß einem der vorstehenden Ansprüche, wobei sich das Isolationssubstrat und die elektrisch leitende Oberfläche in einem Winkel von 90° treffen.

## Revendications

1. Pince électrochirurgicale (20, 100) présentant des effecteurs d'extrémité opposés (22, 44) et une poignée (18, 118) pour effectuer un déplacement des effecteurs d'extrémité l'un par rapport à l'autre, comprenant:
un boîtier (71);
une paire d'électrodes (110, 120) comportant chacune une surface électriquement conductrice (116, 126) et un substrat isolant (111, 121);
**caractérisée en ce que**
les dimensions et/ou la forme géométrique du substrat isolant sont différentes des dimensions et/ou de la forme géométrique de la surface électriquement conductrice afin de réduire la dispersion thermique vers des structures de tissu voisines;
le substrat isolant est réalisé à partir d'une matière ayant un Indice de Poursuite Comparatif d'environ 300 volts à environ 600 volts afin de réduire l'incidence d'amorçage; et
une matière conformable (161) est disposée autour d'un bord périphérique externe de la surface électriquement conductrice et du substrat isolant.

2. Pince selon la revendication 1, dans laquelle le bord périphérique externe d'au moins une surface électriquement conductrice présente un rayon et où l'isolant rencontre la surface électriquement conductrice le long d'un bord de jonction qui se trouve le long du rayon.

3. Pince telle que revendiquée à la revendication 1 ou 2, dans laquelle la matière du substrat isolant est un polyamide ou un polystyrène.

4. Pince selon l'une quelconque des revendications précédentes, dans laquelle le substrat isolant est monté sur la surface électriquement conductrice par surmoulage d'une plaque emboutie.

5. Pince selon l'une quelconque des revendications 1 à 3, dans laquelle le substrat isolant est monté sur la surface électriquement conductrice par surmoulage d'une plaque métallique moulée par injection.

6. Pince selon l'une quelconque des revendications précédentes, dans laquelle la surface électriquement conductrice est relevée par rapport à l'isolant.

7. Pince selon l'une quelconque des revendications précédentes, dans laquelle la surface électriquement conductrice d'au moins une électrode comprend un arrangement pincé (131) et où le substrat isolant s'étend au-delà du pourtour de la surface électriquement conductrice.

8. Pince selon l'une quelconque des revendications précédentes dans laquelle le substrat isolant de chacune des électrodes comporte au moins une interface mécanique pour engager une interface mécanique complémentaire disposée sur l'effecteur d'extrémité correspondant de l'instrument.

9. Pince selon la revendication 8, dans laquelle l'interface mécanique d'au moins un des substrats comporte au moins un goujon d'arrêt (112, 122) et où l'interface mécanique de l'effecteur d'extrémité correspondant comporte au moins une douille complémentaire (41, 43) pour recevoir le goujon d'arrêt.

10. Pince selon l'une quelconque des revendications précédentes, dans laquelle les surfaces électriquement conductrices des éléments de mâchoire opposés coopèrent pour sceller un tissu.

11. Pince selon l'une quelconque des revendications précédentes, dans laquelle les surfaces électriquement conductrices des éléments de mâchoire opposés comprennent des surfaces de cisaillage qui coopèrent pour couper un tissu.

12. Pince selon l'une quelconque des revendications précédentes, dans laquelle le boîtier comprend une extrémité distale fourchue qui délimite deux dents et où chaque dent est fixée de manière amovible à l'un des effecteurs d'extrémité.

13. Pince selon l'une quelconque des revendications précédentes, dans laquelle au moins l'un parmi des effecteurs d'extrémité opposés et les électrodes opposées est tronconique.

14. Pince selon l'une quelconque des revendications précédentes, dans laquelle les effecteurs d'extrémité sont disposés en faisant un angle (α) par rapport à une branche de l'instrument électrochirurgical.

15. Pince selon la revendication 14, dans laquelle l'angle est d'environ soixante degrés à environ soixante-dix degrés.

16. Pince selon l'une quelconque des revendications précédentes, dans laquelle le substrat isolant et la surface électriquement isolante se rencontrent en faisant un angle de quatre-vingt dix degrés.
